Europäisches Patentamt

European Patent Office

Office européen des brevets

(11) Publication number: **0 104 935**
**A2**

# (12) EUROPEAN PATENT APPLICATION

(21) Application number: **83305760.7**

(22) Date of filing: **27.09.83**

(51) Int. Cl.³: **G 01 N 31/08**
**G 01 N 27/30, C 12 M 1/40**

(30) Priority: **28.09.82 US 425155**

(43) Date of publication of application:
**04.04.84 Bulletin 84/14**

(84) Designated Contracting States:
**AT BE CH DE FR GB IT LI LU NL SE**

(71) Applicant: **THE YELLOW SPRINGS INSTRUMENT COMPANY, INC.**
**P.O. Box 279**
**Yellow Springs Ohio 45387(US)**

(72) Inventor: **Johnson, Jay Michael**
**3055 Old Heritage Way**
**Dayton Ohio 45432(US)**

(74) Representative: **Warren, Keith Stanley et al,**
**BARON & WARREN 18 South End Kensington**
**London W8 5BU(GB)**

(54) Liquid chromatograph enzyme electrode detector.

(57) An apparatus and method for detecting a solute in a liquid is provided wherein the liquid is passed through a chromatographic column (12). The effluent of the column (12) is then exposed to an enzyme electrode (20). The output of the electrode is supplied to an appropriate recorder and/or display device (22).

FIG-1

EP 0 104 935 A2

# LIQUID CHROMATOGRAPH ENZYME ELECTRODE DETECTOR

The present invention relates to the detection of a solute in a liquid, and more particularly, to an apparatus and method for detecting components within the effluent of a liquid chromatographic column.

As used herein, the term "liquid chromatography" should be understood to include any means by which component separation of a liquid sample is achieved including thin layer chromatography, separations achieved using tubular cylinders with stationary phase attached to the wall, and the like.

It is well accepted that a good detector for use with a liquid chromatographic column should have high sensitivity, low cell volume, a wide range of response, and a large linear range. Additionally, the detector should be little influenced by experimental parameters, i.e., quantities such as flow rate and temperature. Moreover, the detector should also have long term stability and reliability. Many such detectors are currently commercially available or have been described. These detectors are either responsive to bulk properties of the column effluent, such as those responsive to refractive index, or may be selective detectors responding to particular properties within the analyte of interest, such as flourescence or electrochemical properties. See, for example, J. J. Kirkland (ed.), Modern Practices of Liquid Chromatography (1971) at 95-122.

The use of immobilized enzymes as components of detection systems has also been described. See, e.g., J. Stewart, "Post Column Derivatization Methodology in High Performance Liquid Chromatography (HPLC)," 1 Trends in Anal. Chem. 170, 171 (1982). Typically, the enzyme is immobilized in a glass bead bed reactor or a tubular reactor through which the chromatographic column effluent is pumped. After passage through the reactor, the product of the enzyme reaction can then be detected with a detector such as those mentioned above. In many cases, this approach can provide increased sensitivity and/or selectivity when compared with traditional non-specific detectors.

In order to obtain the highest sensitivity, it is necessary that total conversion of the eluting substrate to product via the enzyme reaction be obtained. Moreover, although complete conversion is not required for detector reliability so long as the enzyme reaction is reproducible, complete conversion is necessary for the response characteristics of the detector system to be independent of the kinetics of the enzyme reaction. Consequently, incomplete conversion results in compromising both the linear response range and the independence of response characteristics from flow rate and temperature. Therefore, it is accepted practice to adjust the residence time in the enzyme reactor so that it is long enough to obtain complete conversion of substrates to products.

Complete conversion and the accompanying increased residence times often can lead to other disadvantages including band broadening due to large void volumes, as well as increased analysis times. Further, it is not possible to recover separated sample components for further analysis, since in order to be detected they are converted to enzyme products. Enzyme products must also not be inhibitors of the enzyme reaction. Moreover, if some dissolved components of the mobile phase were necessary for the enzyme reaction to proceed (such as $O_2$ cofactors or buffering capacity), the necessity for complete conversion could lead to depletion of these components at high substrate concentrations, thereby limiting the linear response range. Also, due to the relatively long residence time, it is important that the product of the enzyme reaction be sufficiently stable to survive to allow detection.

Finally, immobilized enzyme reactors are typically expensive because of the need for high enzyme loading. Such reactors further suffer from the lack of long term stability because the enzyme can wash out of the reactor or plugging of the reactor can occur. In the former case, even longer residence times would be necessary as the reactor aged in order to obtain complete conversion.

What is needed, therefore, is a more appropriate means by which to incorporate immobilized enzyme technology into chromatographic detection such that the benefits of increased sensitivity and selectivity can be realized without sacrificing other features which are important to efficient detection.

The present invention provides a method for detecting a solute in a liquid wherein, according to one aspect of the present invention, the liquid is passed through a chromatographic column. The effluent of the column is then exposed to an enzyme electrode. The output of the electrode is supplied to an appropriate recording and/or display means.

While immobilized enzyme electrodes are well known per se, it has not been heretofore recognized that these electrodes may be used in chromatographic detection. Most enzyme electrodes are configured in such a way that access of substrate to the enzyme is limited. Thus, only a small percentage of substrate, typically on the order of about 1%, is converted to product during a normal analysis. Consequently, under typical chromatographic conditions, the sensitivity one would expect to obtain using these electrodes as detectors would be low when compared to both immobilized reactor systems, wherein the substrate is completely converted to product, or through other methods of detection. Contrary to expectations, in many cases enzyme electrode detection can in fact be much more sensitive than the traditional detection methods.

Additionally, it should be noted that in the development and use of enzyme electrodes, the emphasis has generally been on their selectivity to allow specific analysis on complex samples. In the development of chromatography detectors, on the other hand, the emphasis has been on non-specificity, allowing them to be used to detect many eluted compounds. If chromatography were capable of obtaining perfect resolution of sample components, detector non-specificity would always be preferred. This ideal is of course not the case, particularly when real samples are considered, since sample components often co-elute. Thus, the specificity of enzyme electrode detection can in some cases allow the descrimination of certain co-eluted components. Furthermore, baseline stability is generally greater with the enzyme electrode, due to its more selective nature.

Accordingly, it is an object of the present invention to provide a method and apparatus for detecting a solute in a liquid wherein a liquid chromatographic column is used in combination with an enzyme electrode; to provide such a method and apparatus that is capable of greater sensitivity than presently known methods and apparatus; to provide an apparatus and method having greater independence of the kinetics of the enzyme reaction than presently known methods and apparatus, and thus is more linear and less sensitive to experimental parameters such as temperature and flow rate; to provide a method and apparatus that is not limited by depletion of $O_2$ or other soluble components in the mobile phase; and to provide a method and apparatus wherein a relatively small amount of enzyme may be used and wherein the enzyme is resistent to being washed away, thereby resulting in a much lower cost per test than obtainable with known methods and apparatus.

In order that the invention may be readily understood, reference will now be made to the accompanying drawings, in which:

Fig. 1 is a schematic diagram showing the apparatus of the present invention for detecting a solute in a liquid;

Fig. 2 is a sectional view of the enzyme electrode cell;

Fig. 3 is a sectional view taken generally along line 3-3 in Fig. 2;

Fig. 4 is a sectional view taken generally along line 4-4 in Fig. 3;

Fig. 5a is a chromatogram obtained using the enzyme electrode cell;

Fig. 5b is a chromatogram obtained using the LDC refractometer; and

Figs. 6a and 6b are identical to Figs. 5a and 5b, respectively, with the exception that sensitivity of the recording means is ten times greater.

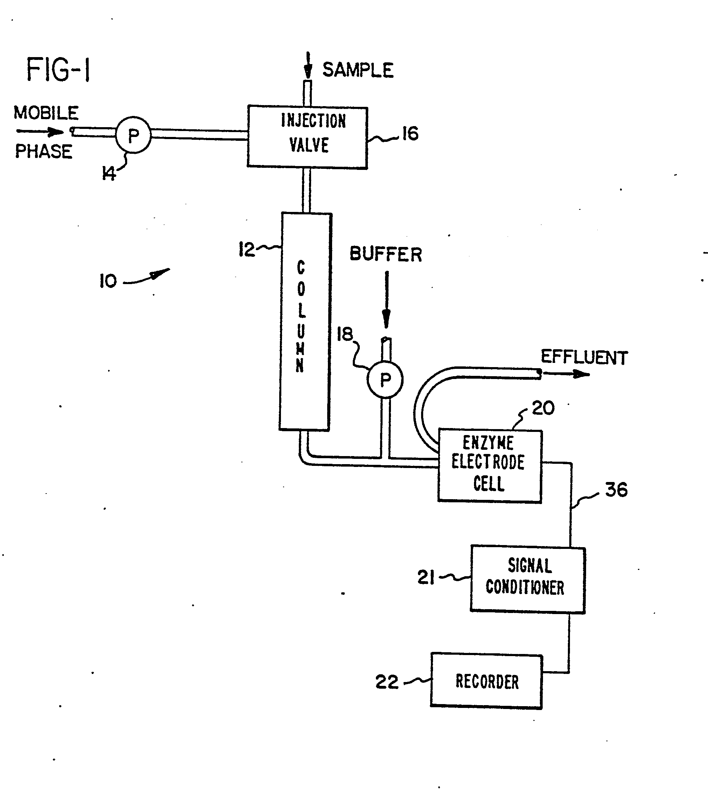

Referring to Fig. 1, an apparatus 10 for detecting a solute in a liquid includes a liquid chromatographic column 12. Column 12 may be any appropriate commercially available chromatographic column, for example, an HPX-87C BIO-RAD column. The mobile phase is supplied to column 12 by a pump 14 through injection valve 16. The sample to be analyzed is introduced into the mobile phase by the injection valve 16, so as to enter column 12.

As the column effluent leaves column 12, an appropriate buffer is added, supplied by pump 18 or other appropriate means. The effluent is then passed through enzyme electrode cell 20, from which it is directed out of the apparatus 10. The output signals from the enzyme electrode cell 20 are provided through an appropriate signal conditioner 21 to a graphic recorder 22 or other data display device.

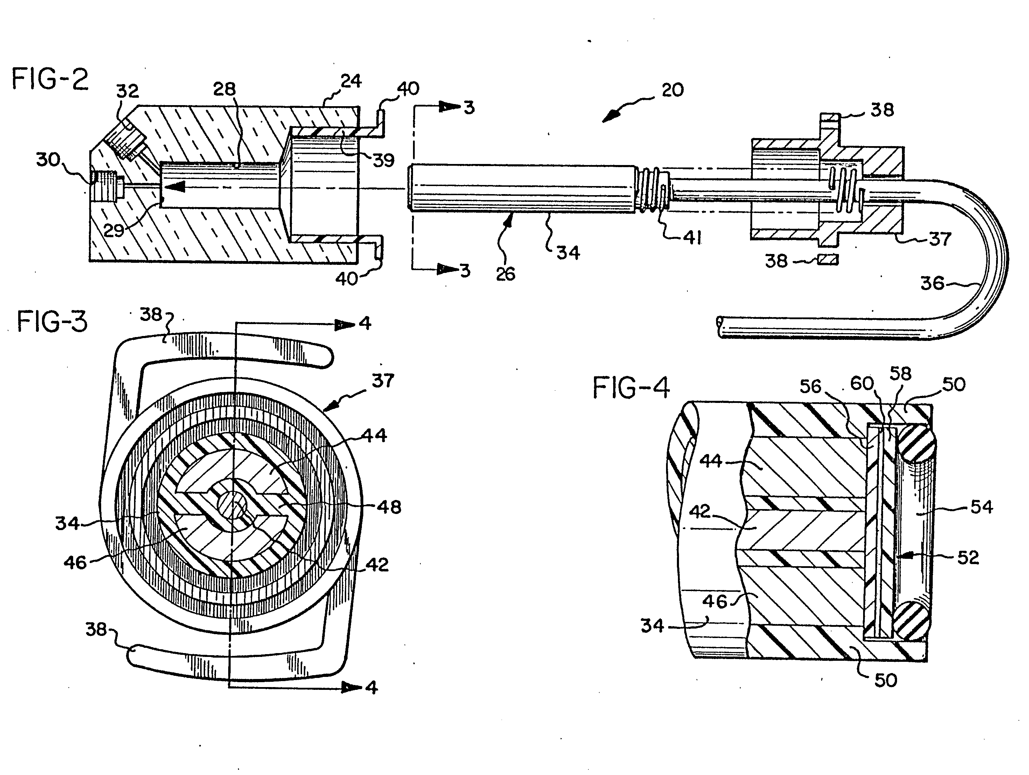

The term "enzyme electrode", as used herein, includes at least two individual electrodes, mounted with an immobilized or entrapped enzyme intimately associated in some manner with the electrodes, and disposed for contact with the column effluent. The preferred enzyme electrode cell 20, essentially a polarographic cell, is shown in greater detail in Fig. 2, and includes cell housing 24 and an insertable cylindrical probe 26. Housing 24 is preferably constructed from clear Lucite, and includes an interior cavity 28 having a base 29. Housing 24 is further provided with an entrance port 30 and an exit port 32, opening into cavity 28 through base 29. Ports 30 and 32 are adapted to cooperate with 1/4-28 fittings, for connection of cell 20 with fluid supply lines as indicated in Fig. 1.

Probe 26 is insertable into cavity 28 of cell housing 24, and includes a solid body 34, preferably constructed from epoxy, to which is connected an electrical cable 36. Cable 36 passes through a cap 37, which is provided with a pair of arms 38 for cooperating with an insert 39, placed into cavity 28 of cell housing 24. Insert 39 includes a pair of tabs 40, which with arms 38 on cap 37, form a bayonet fitting for securing probe 26 in place within housing 24. A spring 41 is disposed about cable 36, with one end adjacent probe body 34, and the other end adjacent cap 37, so that when secured into cell housing 24, probe body 34 is urged by spring 41 against the base 29 of cavity 28.

Referring now to Fig. 3, the face and area adjacent thereto of probe body 34 contains a plurality of electrodes, which are held in place by casting the body 34 about the positioned electrodes. The electrodes extend into body 34 at least slightly beyond its face, and include a central axial platinum electrode 42. A pair of semicircular Ag/AgCl electrodes 44 and 46 are disposed about electrode 42, with the intervening space between the electrodes filled with the epoxy material. Appropriate leads (not shown) are attached to each of the electrodes and extend through probe body 34 to cable 36. The platinum electrode 42 is polarized at +0.7 volts as compared with one of the Ag/AgCl electrodes 44 or 46, and the second Ag/AgCl electrode 44 or 46 is used as an auxiliary electrode.

As seen in Fig. 4, the leading end of probe body 34 includes an annular flange 50. An O-ring mounted enzyme membrane 52 is insertable into the opening formed by flange 50, such that membrane 52 covers the exposed surfaces of electrodes 42, 44 and 46. Mounted to and supporting the membrane 52 is a rubber O-ring 54, selected to be of cross-sectional diameter such that when in place on probe body 34, O-ring 54 extends slightly beyond the end of flange 50. Thus, when probe 26 is inserted into cavity 28 of cell housing 24, O-ring 54 is slightly compressed against cavity base 29, thereby providing a seal about the openings into ports 30 and 32. Thus, the inner diameter and thickness of O-ring 54 defines the test volume of the enzyme electrode cell 20, which is preferably on the order of 5-50 microliters.

In operation of the enzyme electrode cell 20, the mobile phase and dissolved substrates enter the cell 20 through port 30 and exit through port 32. The mobile phase and dissolved substrates, when within cell 20, contact the enzyme membrane 52. Enzyme membrane 52 is a multi-layer semi-permeable membrane that contains at least an immobilized oxidase enzyme, and is described in detail in U.S. Pat. No. 3,979,274, issued September 7, 1976, to Newman, which is hereby incorporated by reference.

Referring to Fig. 4, layer 56 of membrane 52 is adjacent the active phases of electrodes 42, 44 and 46, and is essentially homogeneous silicone, metyl methacrylate or cellulose acetate material, permeable only to small molecules such as $H_2O_2$. Layer 58 is the outer layer which is in contact with the sample to be analyzed. In the preferred embodiment, layer 58 is a 0.03 micron pore size perforated polycarbonate film having a thickness of five microns, a nitrogen flow rate of 25 ml/min/cm$^2$ at 10 psi, and having $6 \times 10^8$ holes/cm$^2$. Typical thicknesses would be 5 microns for layer 58, 1 micron for layer 56, and 1 micron for layer 60, for a total of 7 microns thickness. Layer 60 is the adhesive-enzyme material bonding layers 56 and 58 together.

During their passage through the cell 20, separated sample substrates can diffuse through layer 58 of the enzyme membrane 52 and enter the enzyme layer 60 where they react with $O_2$ in the presence of one or more enzymes to form $H_2O_2$ which diffuses toward the platinum electrode 42 where oxidation of $H_2O_2$ to $O_2$ and $H_2O$ occurs. The use of $H_2O_2$ detection

in an enzyme electrode is well known; see U.S. Patent No. 3,539,455, issued November 10, 1970, to Clark. The peak or integrated current flow obtained between the electrodes 42 and 44 or 46 is thus proportional to substrate concentration. A potential proportional to the current flow is then supplied to recorder 22.

As an alternative approach, the depletion of dissolved $O_2$ due to the enzyme reaction can be measured by the use of suitable electrodes in order to quantify substrates. See, e.g., U.S. Patent No. 3,542,662, issued November 24, 1970, to Hicks et al.

A number of different applications exist for which enzyme electrode detection of chromatographic column substrates is suitable. For example, the apparatus described above may be used to analyze a variety of samples, such as food items, wine, beer, or other organic materials for sugars, alcohols, and the like. Typical substrates that may be detected include glucose and glucose polymers, galactose and galactose polymers, activated alcohols, and primary alcohols such as methanol, ethanol, n-propanol, and n-butanol. Of course, the particular substrate or substrates that may be detected is dependent upon the particular enzyme or enzymes contained in the enzyme membrane 52 of electrode cell 20. Table 1 presents the various preferred enzymes which may be used in the enzyme membrane 52 to perform these and other measurements, along with the particular corresponding columns necessary to perform the required separations.

TABLE 1

| ENZYME USED IN ELECTRODE | SUBSTRATES MEASURED FOR | CHROMATOGRAPHIC COLUMNS |
|---|---|---|
| Glucose Oxidase & Amyloglucosidase (Glucose Polymer) | Glucose, Maltose, Maltotriose, Maltotetrose, etc. | Bio-Rad HPX-42A |
| Galactose Oxidase | Galactose, Lactose, and Galactose Polymers Also Various Activated Alcohols | Bio-Rad HPX-87C |
| Alcohol Oxidase | Methanol, Ethanol, n-Butanol, n-Propanol | Bio-Rad HPX-85H |
| L-Amino Acid Oxidase | L-Amino Acids | Waters Bondapack-alkylphenyl |
| Xanthine Oxidase | Various Purines Pyrimidines and Aldehydes | Whatman Partisil-10 SCX |
| Monoamine Oxidase | Primary, Secondary and Tertiary Amines Catechol Amines Tryptamine Derivatives | Zipax SCX, Vydac SCX, Waters Bondapack-C-18 |
| Diamine Oxidase | Monoamines, Diamines, Polyamines and Amino Acids | Beckman PA-35 |
| Lactate Oxidase | Lactate, Glycolate, Alpha-hydroxyacids | Bio-Rad HPX-87H |

It will be recognized that when used in an enzyme electrode detector, the relative non-specificity of the enzymes is an important asset since it increases the utility of the detector. On the other hand, the combination of the known specificity of the enzyme electrode and the chromatographic retention time allows for a more informative two-dimensional analysis than is obtainable with totally non-specific detection.

To demonstrate the results obtained with the apparatus 10, a specific analysis will be considered. 20 microliters of champagne was injected by valve 16 into column 12, in this case a 30 cm HPX-87C BIO-RAD column thermostated at 85°C. Degassed $H_2O$ was used as the mobile phase, supplied by pump 14, here a Milton Roy Minipump, at a flow rate of 24 ml/hr. In order to provide comparitive results, the effluent from column 12 was initially passed through an LDC refractive index detector (not shown in Fig. 1). The buffer, introduced by pump 18, was a pH 7.3 phospate buffer which also contained ferricyanide. After buffering, the column effluent was passed through the enzyme electrode cell 20, in which the enzyme membrane 52 contained the enzyme galactose oxidase.

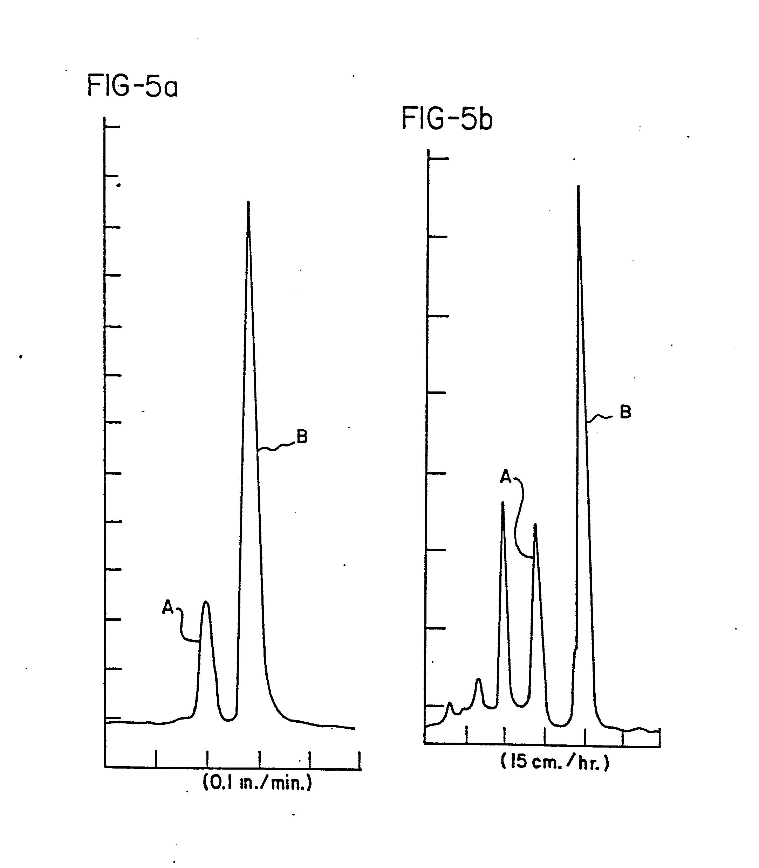

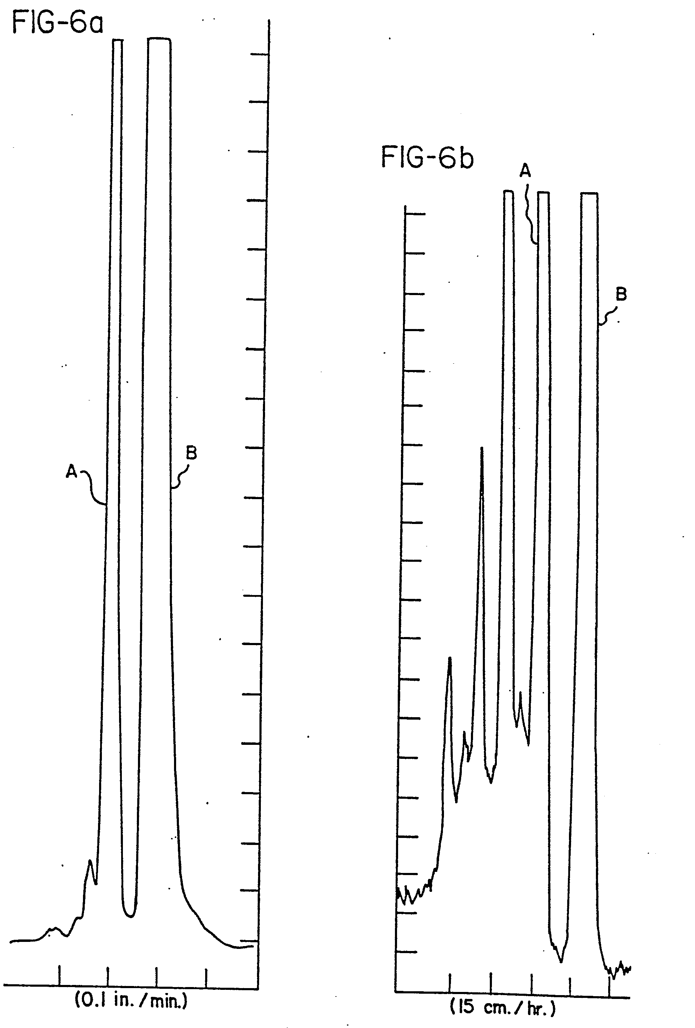

The chromatograms obtained using the enzyme electrode cell are shown on Figs. 5a and 6a, and the LDC refractometer ones are shown in Figs. 5b and 6b, respectively. As will be recognized, the horizontal axes of the chromatograms represent time, and the various peaks of the curves correspond to passage of substrates through the detectors to which the detectors are sensitive. Corresponding peaks of each of the various curves are identified with corresponding letters A and B. Comparison of Fig. 5a, illustrating results with the enzyme electrode cell, and Fig. 5b, showing results with the refractometer, both recorded with recorders at 1 volt full scale, illustrate how enzyme-electrode detection can both simplify the chromatogram obtained and alleviate the elevated and variable base line which is evident in the refractive index chromatogram. The simplification of the chromatogram results from the specificity of the enzyme electrode. This can be an advantage in some cases, since if only the components detectable by the enzyme electrode are of interest, samples can be injected more closely together than if refractive index detection is used, thereby increasing sample throughout. The variable base line of the refractive index chromatogram causes uncertainty in peak quantification, which is significantly diminished in the enzyme-electrode chromatogram.

Figs. 6a and 6b are identical to Figs. 5a and 5b with the exception that sensitivity of the recording means is ten times greater (0.1 volt full scale) in Figs. 6a and 6b than in Figs. 5a and 5b. The elevated base line in the refractive index chromatogram, Fig. 6b, is even more apparent, and it is clear that the noise in the refractive index chromatogram is two to three orders of magnitude greater than the noise in the enzyme-electrode chromatogram, Fig. 6a. This results directly from the fact that refractive index measurements, unlike the case of enzyme-electrode measurements, are very sensitive to hydrodynamic pulsing from the pump used to deliver the mobile phase. Thus, use of refractive index at high sensitivity requires either very expensive pumps in which pulsing is eliminated, electronic means for compensating for pulsing, or a system for pulse dampening. This is not true in the case of enzyme electrodes and thus the signal-to-noise ratio obtainable using the enzyme electrode in a pulsing system can be on the order of 200 to 1000 times greater than with refractive index analysis.

-13-

## CLAIMS

1.      An apparatus for detecting a solute in a liquid, including a chromatographic column (12) and means for introducing said liquid into said column (12), characterized by:

an enzyme electrode (20); and

means (24) for collecting the effluent from said column (12), and for directing said effluent toward and exposing said effluent to said enzyme electrode.

2.      An apparatus as claimed in claim 1, wherein said enzyme electrode includes a polarographic cell (20) having an inlet port (30) and an outlet port (32), said effluent being supplied to said cell (20) at said inlet port (30) and exiting said cell at said outlet port (32) , at least a pair of electrodes (42, 44) exposed within the interior of said cell (20), and a multi-layer semi-permeable membrane (52) having one layer of at least one enzyme, said membrane being disposed within said cell (20) so as to separate said ports (30, 32) from said electrodes (42, 44).

3.      An apparatus as claimed in claim 2, wherein said enzyme includes at least one oxidase.

4.      An apparatus as claimed in claim 2, wherein said enzyme is galactose oxidase.

5.      An apparatus as claimed in claim 1, 2, 3 or 4, further including means (18) for introducing a buffer into said effluent prior to exposure to said enzyme electrode (20).

6.     An apparatus as claimed in claim 3 or 4, wherein said pair of electrodes (42, 44) are adapted for measuring the quantity of $H_2O_2$ produced by exposure of said effluent to said enzyme.

7.     An apparatus as claimed in claim 3 or 4, wherein said electrodes (42, 44) are adapted for measuring the depletion of dissolved $O_2$ from said effluent during exposure of said effluent to said enzyme.

8.     A method of detecting a solute in a liquid, including the step of passing said liquid through a chromatographic column (12); characterized by exposing the effluent of said column (12) to an enzyme electrode (20).

9.     A method as claimed in claim 8, further including the step of mixing a buffer solution with said effluent prior to exposure to said enzyme electrode (20).

10.     A method as claimed in claim 8 or 9, wherein said enzyme electrode (20) includes an oxidase enzyme.

11. A method as claimed in claim 8 or 9 wherein the solute to be detected includes dissolved sugars and alcohols, and said enzyme electrode (20) is a galactose oxidase enzyme electrode.

FIG-1

FIG-2

32  28  24  40  3  20  38

30

39

29  26  34  41  38  37

40  3  38  36

FIG-3

38  4

37

44

48

34  42

46

38

4

FIG-4

56  60  58  50

44

42  54

52

46

34

50

2 / 4

0104935

FIG-5a

FIG-5b

A

B

(0.1 ın./min.)

A

B

(15 cm./hr.)

FIG-6a

A

B

(0.1 in. / min.)

FIG-6b

A

B

(15 cm. / hr.)